# EUROPEAN PATENT SPECIFICATION

(11) **EP 2 907 532 B1**
(45) Date of publication and mention of the grant of the patent: **25.12.2019**
(21) Application number: 15155057.1
(22) Date of filing: 13.02.2015
(51) Int. Cl.: A61L 31/12, A61L 31/14

(54) **Method for forming dual-layer composite material, dual-layer composite material thereby, bio-medical equipment containing the dual-layer composite material**
Verfahren zur Herstellung von zweischichtigem Verbundmaterial, zweischichtiges Verbundmaterial damit, biomedizinisches Gerät mit dem zweischichtigen Verbundmaterial
Procédé de formation d'un matériau composite bicouche, matériau composite bicouche, équipement biomédical contenant le matériau composite bicouche

(30) Priority: 14.02.2014 US 201461940257 P; 05.02.2015 TW 104103831
(43) Date of publication of application: 19.08.2015
(73) Proprietor: Han Biomedical Inc., 221 New Taipei City (TW)
(72) Inventor: Yen, Hsiao-Cheng, 103 Taipei City (TW); Chang, Chih-Long, 103 Taipei City (TW)
(74) Representative: Fuchs Patentanwälte Partnerschaft mbB

(56) References cited:
- US-A- 5 201 745
- US-A1- 2002 131 933
- US-A1- 2010 040 685
- US-B1- 6 596 304

## Description

The present disclosure relates to a bio-medical material, and in particular it relates to a dual-layer composite material and the formation method thereof.

Fibrous tissues which result in adhesion usually connect between two tissues, and usually cross space of a body cavity, such as the peritoneal cavity. Generally, the causes of postsurgical adhesion may include tissue incisions, handling of internal organs, drying out of internal organs and tissues, contact of internal tissues with foreign materials, such as gauze, surgical gloves, stitches, etc., and blood or blood clots that were not rinsed out during surgery.

Operations on the lower abdomen and pelvis include bowel and gynecological surgeries, and the risk of postsurgical adhesion is high. While the "connections" that result from such adhesion may not cause problems, they may cause tissues or organs at the two ends thereof to become twisted or pulled away from their normal positions. This can negatively affect their normal physiological functioning, and cause pain and medical complications. US 6 596 304 B1 discloses biodegradable bicomposite haemostatic surgical patches with a collagenic layer on a high porosity fibrous polymeric compress layer. US 2010/040685 A1 discloses a collagen-based matrix, useful e.g. as restorative material, prepared by spreading and lyophilizing an atelocollagen dispersion, eventually in admixture with hyaluronic acid, on releasable plates to form the porous and dense membranes, overlaying and crosslinking the membranes. US 5 201 745 A discloses a patch for use in visceral surgery, made from a biomaterial comprising two superimposed layers of a collagen which are joined closely together, e.g. one porous layer of a fibrous collagen and a film layer of a collagen or gelatin, and the patch can be made by pouring a solution of the film collagen onto a fibrous layer of the appropriate shape.

Therefore, a novel anti-adhesion material which can be used to promote postsurgical wound healing is needed.

The present disclosure provides a method for forming a dual-layer composite material, comprising: coating a barrier-film layer-forming material onto a surface of a porous scaffold layer to form a dual-layer intermediate product; and drying the dual-layer intermediate product to form a dual-layer composite material which comprises the porous scaffold layer and a barrier film layer, wherein the porous scaffold layer and the barrier film layer are inseparable from each other, and wherein a method for forming the porous scaffold layer comprises: mixing at least one first biodegradable polymer with a first solvent under a stirring speed of 3500-12000 rpm, at 4-10°C, for 90-180 minutes, to form a slurry, wherein the at least one first biodegradable polymer is selected from a group consisting of collagen, gelatin, chitosan, and a combination of collagen, gelatin or chitosan, and hyaluronic acid; placing the slurry into a mold and freezing the slurry; performing a lyophilization procedure on the slurry to remove water molecules and obtain a scaffold body; and performing a vacuum heating procedure on the scaffold body to dehydrate and crosslink the scaffold body to form the porous scaffold layer. The method for forming the barrier-film layer-forming material comprises: mixing a second biodegradable polymer with a second solvent and then letting it stand to form a gel, wherein the second biodegradable polymer is selected from a group consisting of collagen, gelatin, chitosan and hyaluronic acid; and stirring the gel to a homogeneous stage to form the barrier-film layer-forming material.

The present disclosure also provides a dual-layer composite material, wherein the dual-layer composite material is a biomedical material, which is formed by the method for forming the dual-layer composite material mentioned above. The dual-layer composite material is in the form of a membrane or in the form of a 3-dimensional shape comprising a hollow part or channel with at least one opening.

The present disclosure further provides bio-medical equipment which comprises the dual-layer composite material formed by the method for forming the dual-layer composite material mentioned above.

The present disclosure also provides a dual-layer composite material, comprising: a porous scaffold layer having the effect of accelerating wound-healing and/or tissue regeneration; and a barrier film layer formed on a surface of the porous scaffold layer, having the effect of preventing tissue-adhesion, wherein the porous scaffold layer and the barrier film layer are inseparable from each other, and wherein porous scaffold layer is formed by at least one first biodegradable polymer, and the at least one first biodegradable polymer is selected from a group consisting of collagen, gelatin, chitosan, and a combination of collagen, gelatin or chitosan, and hyaluronic acid, and wherein the barrier film layer is formed by a second biodegradable polymer, and the second biodegradable polymer is selected from a group consisting of collagen, gelatin, chitosan and hyaluronic acid.

Moreover, the present disclosure provides bio-medical equipment comprising the foregoing dual-layer composite material.

A detailed description is given in the following embodiments with reference to the accompanying drawings.

The present disclosure can be more fully understood by reading the subsequent detailed description and examples with references made to the accompanying drawings, wherein:
FIG. 1 shows the structure of the dual-layer composite material of the present disclosure;
FIG. 2 shows the dual-layer composite material of the present disclosure in the form of a membrane;
FIG. 3 shows an oval-shaped embodiment for the dual-layer composite material of the present disclosure in the form of a 3-dimensional shape comprising a hollow part or channel with at least one opening;
FIG. 4 shows another oval-shaped embodiment for the dual-layer composite material of the present disclosure in the form of a 3-dimensional shape comprising a hollow part or channel with at least one opening;
FIGS. 5A and 5B show a tube-shaped embodiment for the dual-layer composite material of the present disclosure in the form of a 3-dimensional shape comprising a hollow part or channel with at least one opening, wherein FIG. 5A is a front view, and FIG. 5B is a cross-sectional view;
FIGS. 6A and 6B show another tube-shaped embodiment for the dual-layer composite material of the present disclosure in the form of a 3-dimensional shape comprising a hollow part or channel with at least one opening, wherein FIG. 6A is a front view, and FIG. 6B is a cross-sectional view;
FIGS. 7A to 7D show a funnel-shaped embodiment for the dual-layer composite material of the present disclosure in the form of a 3-dimensional shape comprising a hollow part or channel with at least one opening. FIG. 7A is a front view, FIG. 7B is a longitudinal section view, FIG. 7C is a top view and FIG. 7D is a bottom view; and
FIGS. 8A to 8D show another funnel-shaped embodiment for the dual-layer composite material of the present disclosure in the form of a 3-dimensional shape comprising a hollow part or channel with at least one opening. FIG. 8A is a front view, FIG. 8B is a longitudinal section view, FIG. 8C is a top view and FIG. 8D is a bottom view.

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are shown schematically in order to simplify the drawing.

In one embodiment, the present disclosure provides a method for forming a dual-layer composite material that has the effect of accelerating wound-healing and/or tissue regeneration while at the same time preventing tissue-adhesion.

The method for forming a dual-layer composite material of the present disclosure comprises the following steps:

First, a barrier-film layer-forming material is coated onto a surface of a porous scaffold layer to form a dual-layer intermediate product.

Then, the dual-layer intermediate product is dried to form a dual-layer composite material which comprises the porous scaffold layer and a barrier film layer. The preceding dual-layer intermediate product may be dried at 15-30°C. In one embodiment, the dual-layer intermediate product may be dried at a room temperature. Moreover, the dual-layer intermediate product may be dried for 12-24 hours. In one embodiment, the dual-layer intermediate product may be dried for 24 hours. In one specific embodiment, the dual-layer intermediate product may be dried at a room temperature for 24 hours to form the dual-layer composite material.

In the dual-layer composite material formed by the preceding method of the present disclosure, the porous scaffold layer has the effect of accelerating wound-healing and/or tissue regeneration and the barrier film layer has the effect of preventing tissue-adhesion, and the porous scaffold layer and the barrier film layer are inseparable from each other.

Furthermore, a method for forming the porous scaffold layer which is mentioned in the method for forming a dual-layer composite material of the present disclosure, comprises the following steps.

First, at least one first biodegradable polymer is mixed with a first solvent at a low temperature, under a high stirring speed to form a slurry. The at least one first biodegradable polymer mentioned above may account for 1.0-2.0 wt% of the slurry.

The at least one first biodegradable polymer is mixed with the first solvent under a stirring speed of 3500-12000 rpm, at 4-10°C, for 90-180 minutes, to form the slurry. Furthermore, in one embodiment, the at least one first biodegradable polymer is mixed with the first solvent under a stirring speed of 10500 rpm, at about 5°C, for about 90 minutes, to form the slurry.

The of at least one first biodegradable polymer used in the method for forming the porous scaffold layer mentioned above is selected from collagen, gelatin, chitosan, a combination of collagen, gelatin or chitosan, and hyaluronic acid. In one embodiment, the at least one first biodegradable polymer may be collagen, such as type I collagen. Moreover, the at least one first biodegradable polymer may be a combination of collagen, gelatin or chitosan, and hyaluronic acid, such as a combination of collagen and hyaluronic acid.

In the embodiment in which the at least one first biodegradable polymer may be a combination of collagen, gelatin or chitosan, and hyaluronic acid, a weight ratio of the collagen, gelatin or chitosan to the hyaluronic acid may be 90-99.9:10-0.1. For example, a weight ratio of the collagen to the hyaluronic acid may be about 93-94:7-6.

In addition, in the embodiment, in which the at least one first biodegradable polymer may be a combination of collagen, gelatin or chitosan, and hyaluronic acid, before the step of mixing at least one first biodegradable polymer with a first solvent at a low temperature, under a high stirring speed, to form a slurry, the method for forming the porous scaffold layer may further comprise mixing the collagen, gelatin or chitosan with the first solvent at a low temperature, under a high stirring speed, previously, to form a mixture and then pouring a solution of the hyaluronic acid into the mixture.

The step of mixing the collagen, gelatin or chitosan with the first solvent at a low temperature, under a high stirring speed, previously is mixing the collagen, gelatin or chitosan with the first solvent under a stirring speed of 3500-12000 rpm, at 4-10°C, for 90-180 minutes, previously, to form a mixture.

Furthermore, the solution of the hyaluronic acid may be formed by dissolving the hyaluronic acid in a solvent. Examples of solvent which is suitable for dissolving the hyaluronic acid may comprise water, acetic acid, isopropanol, etc. In one embodiment, the acetic acid is 0.05 M acetic acid.

In addition, the first solvent used in the method for forming the porous scaffold layer mentioned above may comprise water, isopropanol or acetic acid. In one embodiment, the first solvent used in the method for forming the porous scaffold layer mentioned above is isopropanol, and the isopropanol may be about 10-20% isopropanol, such as 10% isopropanol. In another embodiment, the first solvent used in the method for forming the porous scaffold layer mentioned above is acetic acid, and the acetic acid may be about 0.02-0.05 M acetic acid, such as 0.05 M acetic acid.

After the at least one first biodegradable polymer is mixed with the first solvent to form the slurry, the formed slurry is placed into a mold and frozen. In this step, the slurry may be frozen at -40 to -20°C. In one embodiment, the slurry may be frozen at about -30°C.

Next, after the slurry is placed into a mold and frozen, a lyophilization procedure is performed on the slurry to remove water molecules and obtain a scaffold body. The pressure in the preceding lyophilization procedure is 3.99 - 26.6 Pa (30-200 mTorr), such as 26.6 Pa (200 mTorr), but is not limited thereto. Moreover, the lyophilization procedure may comprise at -40 to -20°C, freezing the slurry for 3-6 hours, then at -40 to 10°C, freezing the slurry for 12-16 hours, and after that placing the slurry at 20 to 30°C, for 3-6 hours, but is not limited thereto. In one embodiment, the pressure in the preceding lyophilization procedure may be 26.6 Pa (200 mTorr), and in this embodiment, the lyophilization procedure may comprise at about -30°C, freezing the slurry for about 3-6 hours, then at about 0°C, freezing the slurry for about 12-16 hours, and after that placing the slurry at about 30°C, for about 3-6 hours.

Furthermore, after the step of performing a lyophilization procedure on the slurry to remove water molecules and obtain a scaffold body, a vacuum heating procedure is performed on the obtained scaffold body to dehydrate and crosslink the scaffold body to form the porous scaffold layer mentioned above. The pressure in the vacuum heating procedure may be 3.99 - 26.6 Pa (30-200 mTorr), such as about 26.6 Pa (200 mTorr), but is not limited thereto. In addition, the temperature in the vacuum heating procedure may be 80-110°C, but is not limited thereto. Furthermore, time for the vacuum heating procedure may be 12-24 hours, such as about 24 hours, but is not limited thereto. In one specific embodiment, the pressure in the vacuum heating procedure is about 26.6 Pa (200 mTorr), the temperature in the vacuum heating procedure is about 80-110°C, and time for the vacuum heating procedure is about 24 hours.

In addition, in one embodiment, the method for forming the porous scaffold layer in addition to the steps mentioned above, between the step of mixing at least one first biodegradable polymer with a first solvent at a low temperature, under a high stirring speed to form a slurry and the step of placing the slurry into a mold and freezing the slurry, may further comprise a step of removing gas in the slurry. There is no specific limitation for the manner for removing gas in the slurry. In one embodiment, gas in the slurry may be removed by vacuum heating.

Moreover, a method for forming the barrier-film layer-forming material which is mentioned in the method for forming a dual-layer composite material of the present disclosure, comprises the following steps.

First, a second biodegradable polymer is mixed with a second solvent and then left to stand to form a gel. The second biodegradable polymer may account for 3-10% w/v of the gel.

Examples of second biodegradable polymer which is suitable for being used in the method for forming the barrier-film layer-forming material mentioned above include collagen, gelatin, chitosan, hyaluronic acid, etc. In one embodiment, the second biodegradable polymer may be collagen, such as type I collagen.

In addition, the second solvent used in the method for forming the barrier-film layer-forming material mentioned above may comprise water or acetic acid. In one embodiment, the second solvent used in the method for forming the porous scaffold layer mentioned above is acetic acid, and the acetic acid may be about 0.1-0.5 M acetic acid, such as 0. 5 M acetic acid.

Next, after forming the gel, the gel is stirred to a homogeneous stage to form the barrier-film layer-forming material.

Moreover, in one embodiment, the method for forming the barrier-film layer-forming material in addition to the steps mentioned above, after the step of stirring the gel to a homogeneous stage, may further comprise a step of removing bubbles in the gel.

In another embodiment, the present disclosure provides a dual-layer composite material, which is formed by any of the methods for forming a dual-layer composite material of the present disclosure, and the mentioned methods for forming a dual-layer composite material may also comprise various aforementioned methods for forming the porous scaffold layer and various aforementioned methods for forming the barrier-film layer-forming material.

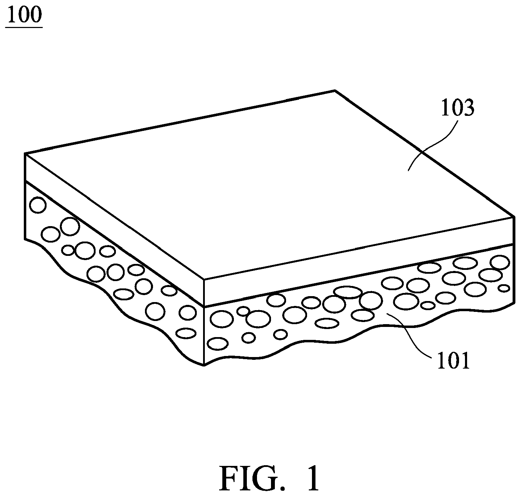

The dual-layer composite material formed by the method for forming a dual-layer composite material of the disclosure has a structure shown in FIG. 1. FIG. 1 shows that dual-layer composite material of the present disclosure 100 has a porous scaffold layer 101 and a barrier film layer 103, wherein the barrier film layer 103 is formed on a surface of the porous scaffold layer 101.

The dual-layer composite material of the present disclosure has the effect of accelerating wound-healing and/or tissue regeneration while at the same time preventing tissue-adhesion. The dual-layer composite material of the present disclosure can be applied in bio-medical use, for example, the dual-layer composite material of the present disclosure can be applied to postsurgical wounds. Furthermore, the direction for use of the dual-layer composite material of the present disclosure is attaching the porous scaffold layer of the dual-layer composite material to a wound.

The porous scaffold layer of the dual-layer composite material of the present disclosure has the effect of accelerating wound-healing and/or tissue regeneration, and can be degraded naturally after the wound has healed. The porous scaffold layer mentioned above is a scaffold structure, and a pore size of the porous scaffold layer may be about 100-500 µm.

The barrier film layer of the dual-layer composite material of the present disclosure has the effect of preventing tissue-adhesion. When the dual-layer composite material of the present disclosure is applied to a wound, the barrier film layer will become a gel within 24 to 48 hours, and can be slowly resorbed and excreted from the body in less than 28 days. The foregoing barrier film layer is a non-porous layer, and the barrier film layer has the property of separating from the injury site and the function of providing a proper interface between the wound and other areas, attenuating the inflammatory signaling, etc., but is not limited thereto.

The dual-layer composite material of the present disclosure may be in the form of a membrane or in the form of a 3-dimensional shape comprising a hollow part or channel with at least one opening.

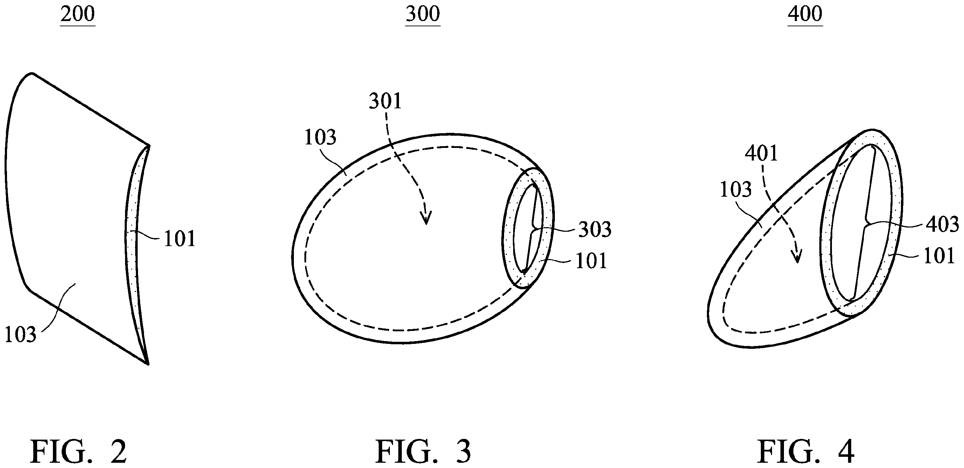

FIG. 2 shows the dual-layer composite material of the present disclosure 200 in the form of a membrane which has a porous scaffold layer 101 and a barrier film layer 103.

The dual-layer composite material of the present disclosure may comprise a 3-dimensional shape that may be for example, a globe shape, an oval-shape, a tube shape, a funnel shape, a cone shape, . In one embodiment, in the dual-layer composite material of the present disclosure in the form of a 3-dimensional shape comprising a hollow part or channel with at least one opening, the porous scaffold layer is located outside of the 3-dimensional shape while the barrier film layer is located inside of the 3-dimensional shape and encompasses the hollow part or channel. In another embodiment, the barrier film layer is located outside of the 3-dimensional shape while the porous scaffold layer is located inside of the 3-dimensional shape and encompasses the hollow part or channel.

FIG. 3 shows an oval-shaped embodiment for the dual-layer composite material of the present disclosure in the form of a 3-dimensional shape comprising a hollow part or channel with at least one opening. In the oval-shaped dual-layer composite material comprising a hollow part or channel with at least one opening of the present disclosure 300 of FIG. 3, the barrier film layer 103 is located outside of the oval-shaped dual-layer composite material of the present disclosure while the porous scaffold layer 101 is located inside of the oval-shaped dual-layer composite material of the present disclosure 300 and encompasses the hollow part 301 and the opening 303.

FIG. 4 shows another oval-shaped embodiment for the dual-layer composite material of the present disclosure in the form of a 3-dimensional shape comprising a hollow part or channel with at least one opening. In the oval-shaped dual-layer composite material comprising a hollow part or channel with at least one opening of the present disclosure 400 of FIG. 4, the barrier film layer 103 is located outside of the oval-shaped dual-layer composite material of the present disclosure while the porous scaffold layer 101 is located inside of the oval-shaped dual-layer composite material of the present disclosure 400 and encompasses the hollow part 401 and the opening 403.

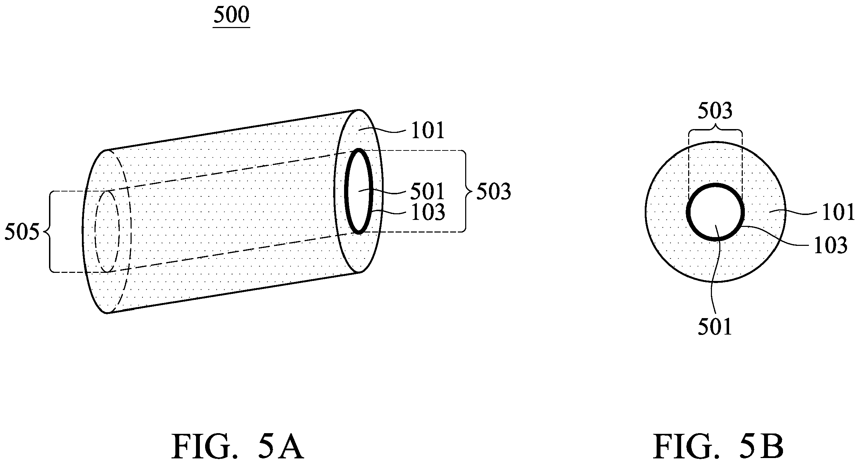

FIGS. 5A and 5B show a tube-shaped embodiment for the dual-layer composite material of the present disclosure in the form of a 3-dimensional shape comprising a hollow part or channel with at least one opening. FIG. 5A is a front view, and FIG. 5B is a cross-sectional view. In the tube-shaped dual-layer composite material comprising a hollow part or channel with at least one opening of the present disclosure 500 of FIGS. 5A and 5B, the porous scaffold layer 101 is located outside of the tube-shaped dual-layer composite material of the present disclosure while the barrier film layer 103 is located inside of the tube-shaped dual-layer composite material of the present disclosure 500 and encompasses the channel 501, the opening 503, and the opening 505.

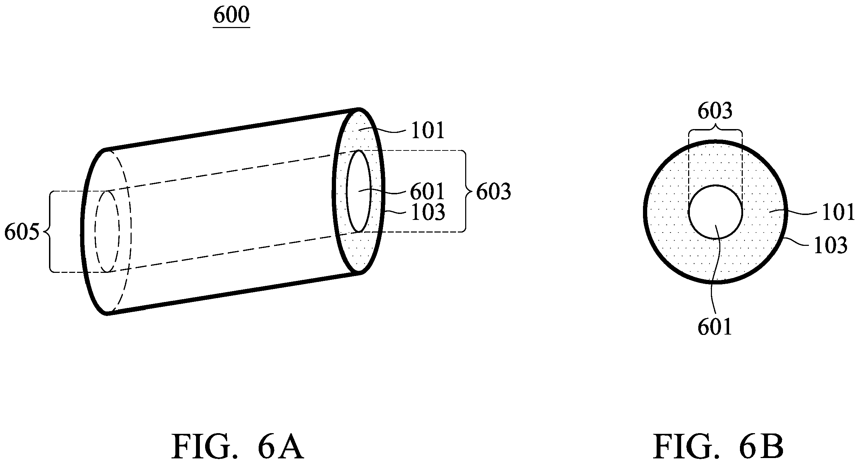

FIGS. 6A and 6B show another tube-shaped embodiment for the dual-layer composite material of the present disclosure in the form of a 3-dimensional shape comprising a hollow part or channel with at least one opening. FIG. 6A is a front view, and FIG. 6B is a cross-sectional view. In the tube-shaped dual-layer composite material comprising a hollow part or channel with at least one opening of the present disclosure 600 of FIGS. 6A and 6B, the barrier film layer 103 is located outside of the tube-shaped dual-layer composite material of the present disclosure while the porous scaffold layer 101 is located inside of the tube-shaped dual-layer composite material of the present disclosure 600 and encompasses the channel 601, the opening 603, and the opening 605.

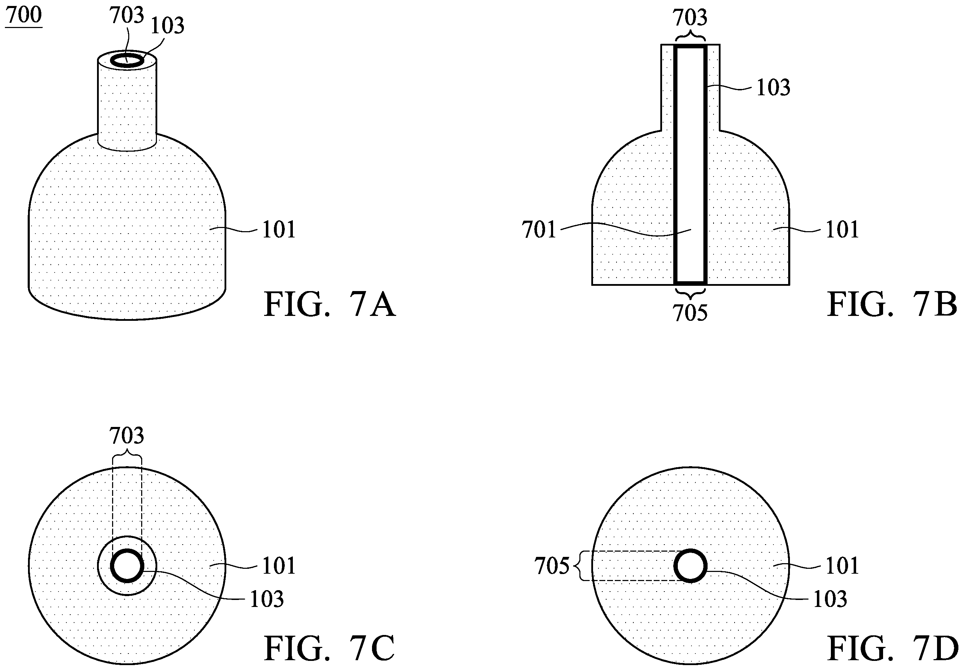

FIGS. 7A to 7D show a funnel-shaped embodiment for the dual-layer composite material of the present disclosure in the form of a 3-dimensional shape comprising a hollow part or channel with at least one opening. FIG. 7A is a front view, FIG. 7B is a longitudinal section view, FIG. 7C is a top view and FIG. 7D is a bottom view. In the funnel-shaped dual-layer composite material comprising a hollow part or channel with at least one opening of the present disclosure 700 of FIGS. 7A to 7D, the porous scaffold layer 101 is located outside of the funnel-shaped dual-layer composite material of the present disclosure while the barrier film layer 103 is located inside of of the funnel-shaped dual-layer composite material of the present disclosure 700 and encompasses the channel 701, the opening 703, and the opening 705. In one embodiment, the funnel-shaped dual-layer composite material comprising a hollow part or channel with at least one opening of the present disclosure 700 can be applied to a postsurgical wound treatment for a uterine cervix operation.

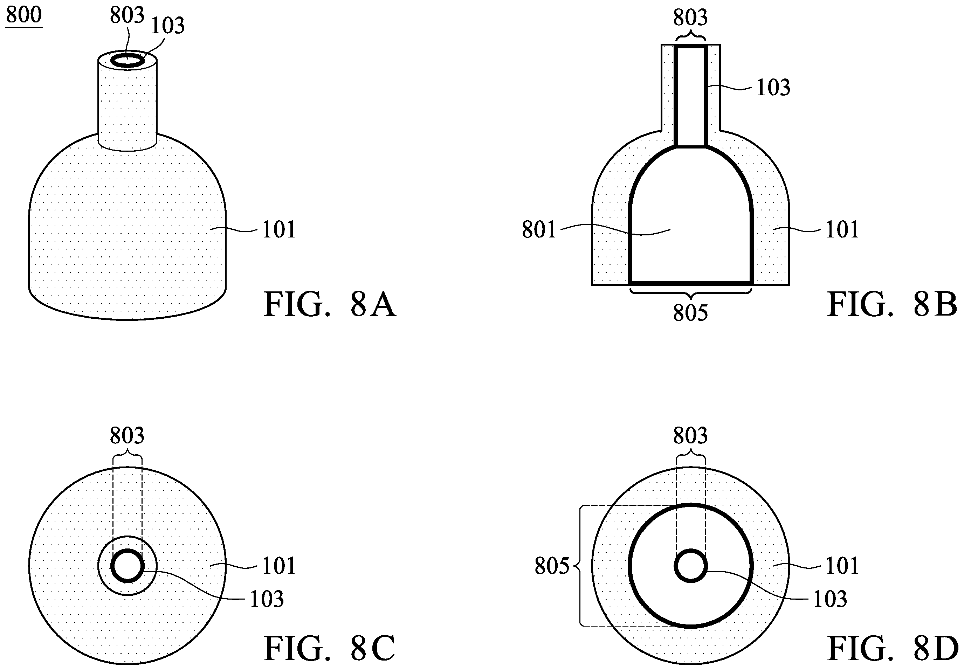

FIGS. 8A to 8D show another funnel-shaped embodiment for the dual-layer composite material of the present disclosure in the form of a 3-dimensional shape comprising a hollow part or channel with at least one opening. FIG. 8A is a front view, FIG. 8B is a longitudinal section view, FIG. 8C is a top view and FIG. 8D is a bottom view. In the funnel-shaped dual-layer composite material comprising a hollow part or channel with at least one opening of the present disclosure 800 of FIGS. 8A to 8D, the porous scaffold layer 101 is located outside of the funnel-shaped dual-layer composite material of the present disclosure while the barrier film layer 103 is located inside of the funnel-shaped dual-layer composite material of the present disclosure 800 and encompasses the funnel-shaped hollow part 801, the opening 803 at the neck of the funnel-shaped hollow part, and the opening 805 at the bottom of the funnel-shaped hollow part. In one embodiment, the funnel-shaped dual-layer composite material comprising a hollow part or channel with at least one opening of the present disclosure 800 can be applied to a postsurgical wound treatment for a uterine cervix operation.

In another embodiment, the present disclosure further provides bio-medical equipment which comprises the dual-layer composite material formed by the method for forming a dual-layer composite material of the present disclosure mentioned above.

In addition, in one embodiment, the present disclosure also provides a dual-layer composite material.

The foregoing dual-layer composite material of the present disclosure comprises a porous scaffold layer and a barrier film layer formed on a surface of the porous scaffold layer, wherein the porous scaffold layer and the barrier film layer are inseparable from each other.

The thickness of the porous scaffold layer mentioned above may be about 0.5-5.0 mm. Furthermore, the thickness of the barrier film layer may be about 0.05-0.5 mm.

The porous scaffold layer of the dual-layer composite material of the present disclosure has the effect of accelerating wound-healing and/or tissue regeneration. Moreover, the porous scaffold layer of the dual-layer composite material of the present disclosure can be degraded naturally after the wound has healed. The foregoing porous scaffold layer is a scaffold structure, and a pore size of the porous scaffold layer may be about 100-500 µm.

The barrier film layer of the dual-layer composite material of the present disclosure mentioned above has the effect of preventing tissue-adhesion. The foregoing barrier film layer is a non-porous layer, and the barrier film layer has the property of separating from the injury site and the function of providing a proper interface between the wound and other areas, attenuating the inflammatory signaling, etc., but is not limited thereto. When the dual-layer composite material of the present disclosure is applied to a wound, the barrier film layer will become a gel within 24 to 48 hours, and can be slowly resorbed and excreted from the body in less than 28 days.

The preceding porous scaffold layer is formed by at least one first biodegradable polymer suitable for forming the porous scaffold layer mentioned above selected from collagen, gelatin, chitosan, a combination of collagen, gelatin or chitosan, and hyaluronic acid. In one embodiment, the at least one first biodegradable polymer may be collagen, such as type I collagen. Moreover, in another embodiment, the at least one first biodegradable polymer may be a combination of collagen and hyaluronic acid.

Moreover, the preceding barrier film layer is formed by a second biodegradable polymer. Examples of second biodegradable polymer which is suitable for forming the barrier film layer mentioned above include collagen, gelatin, chitosan, and hyaluronic acid.

The dual-layer composite material of the present disclosure may be in the form of a membrane or in the form of a 3-dimensional shape comprising a hollow part or channel with at least one opening (for example, referring to FIGS 2 to 7D).

In further another embodiment, the present disclosure further provides bio-medical equipment comprising the foregoing dual-layer composite material.

### EXAMPLES

### A. Preparation of different dual-layer composite materials of the present disclosure

### Example 1

### Preparation of a dual-layer composite material having a porous collagen scaffold layer and a hyaluronic acid barrier film layer

The method for preparing a dual-layer composite material having a porous collagen scaffold layer and a hyaluronic acid barrier film layer is described below.
1. 4.5 g type I collagen was added into the blender containing 300 ml 10% isopropyl Alcohol, then was blended under 5.0°C, under 10500 rpm for 90 minutes to obtain a slurry.
2. The slurry was placed into a vacuum oven to be vacuumed to remove the gas in the slurry.
3. 10 ml of the slurry was poured into a mold with the dimension of 6x6 cm² and placed in a freeze-dryer to be frozen at -30°C.
4. The vacuum pump of the freeze-dryer was started to keep the pressure of the freeze-dryer chamber under 26.6 Pa (200mTorr).
5. The slurry in the freeze-dryer was maintained at -30°C for 3 hours.
6. The temperature of the freeze-dryer was raised to 0°C and the slurry was maintained at this temperature for 12 hours.
7. The temperature of the freeze-dryer was raised to 30°C and the slurry was maintained at this temperature for 6 hours.
8. The vacuum pump of the freeze-dryer was stopped and the formed scaffold body was taken out of the vacuum chamber.
9. The scaffold body was placed in a the vacuum oven and the vacuum pump of was started to keep the pressure under 26.6 Pa (200 mTorr), and then the temperature of the vacuum oven was raised to 105°C to maintain the scaffold body at this temperature for 24 hours.
10. 30 ml of water and 2.0 g of hyaluronic acid were added into a 50-ml syringe, and then left to stand overnight to form a gel.
11. Water was added into the syringe to the total volume of the content in the syringe to be 50 ml.
12. The formed hyaluronic acid gel was stirred tenderly to a homogeneous stage.
13. The syringe was vertically placed to let the bubbles depart from the hyaluronic acid gel surface.
14. 20 ml of the hyaluronic acid gel was added into a plastic mold with the dimension of 6x6 cm² and the surface of the hyaluronic acid gel waited to be flattened.
15. The scaffold body was placed onto the surface of the hyaluronic acid gel and air-dried under room temperature for 24 hours in a laminar flow.

### Example 2

### Preparation of a dual-layer composite material having a porous collagen/hyaluronic acid scaffold layer and a collagen barrier film layer

The method for preparing a dual-layer composite material having a porous collagen/hyaluronic acid scaffold layer and a collagen barrier film layer is described below.
1. 4.5 g type I collagen was added into the blender containing 250 ml 0.05M acetic acid, then was blended at 5.0°C, under 10500 rpm for 90 minutes to obtain a collagen slurry.
2. 0.15 g hyaluronic acid was added into 50 ml of 0.05M acetic acid and stirred to be well-dissolved.
3. The hyaluronic acid solution was added into the blender containing the foregoing collagen slurry and blended at 5.0°C, under 10500 rpm for 90 minutes to obtain a collagen/hyaluronic acid slurry.
4. The collagen/hyaluronic acid slurry was placed into a vacuum oven to be vacuumed to remove the gas in the collagen/hyaluronic acid slurry.
5. 10 ml of the collagen/hyaluronic acid slurry was poured into a mold with the dimension of 6x6 cm² and placed in a freeze-dryer to be frozen at -30°C.
6. The vacuum pump of the freeze-dryer was started to keep the pressure of the freeze-dryer chamber under 26.6 Pa (200mTorr).
7. The slurry in the freeze-dryer was maintained at -30°C for 3 hours.
8. The temperature of the freeze-dryer was raised to 0°C and the slurry was maintained at this temperature for 12 hours.
9. The temperature of the freeze-dryer was raised to 30°C and the slurry was maintained at this temperature for 4 hours.
10. The vacuum pump of the freeze-dryer was stopped and the formed collagen/hyaluronic acid scaffold body was taken out of the vacuum chamber.
11. The collagen/hyaluronic acid scaffold body was placed in a the vacuum oven and the vacuum pump of was started to keep the pressure under 26.6 Pa (200 mTorr), and then the temperature of the vacuum oven was raised to 105°C to maintain the scaffold body at this temperature for 24 hours.
12. 30 ml of 0.5 M acetic acid and 4.0 g of collagen were added into a 50-ml syringe, and then left to stand overnight to form a gel.
13. 0.5 M acetic acid was added into the syringe to the total volume of the content in the syringe to be 50 ml.
14. The formed collagen gel was stirred tenderly to a homogeneous stage.
15. The syringe was vertically placed to let the bubbles depart from the collagen gel surface.
16. 20 ml of the collagen gel was added into a plastic mold with the dimension of 6x6 cm² and the surface of the collagen gel waited to be flattened.
17. The collagen/hyaluronic acid scaffold body was placed onto the surface of the collagen gel and air-dried under room temperature for 24 hours in a laminar flow.

### B. Animal experiments

### (a) Material and method

1. Electronic equipment: weight scale, electric razor, electro-coagulator (60 W)
2. Anesthetic: Zoletil 50: 0.025 ml/100 g body weight (B.W.) + xylazine (Rompun): 0.025 ml/100 g body weight (SD Rat)
3. Solution: ethanol, sterile saline solution, tincture of iodine, formalin fixing solution.
4. General material: paper towel, 50 ml centrifuge tube, plastic dropper, gauze, trash bag, 1 ml-syringe
5. Suture: 3-0 black silk sutures

Surgical instrument: surgical scissors, dressing forceps with single hook, needle holders, hemostatic forceps, scalpel

### (b) Experimental animals

### 1. Rat

Strain: Sprague-Dawley (SD) Rats
Sex: Female
Age: 12-14 weeks
Body weight: 210-260 g

### (c) Experimental methods and results

### 1. Method

1.1 The rats were divided into three groups, wherein the first group was a control group, the second group was treated with a commercial product, Seprafilm®, and the third group was treated with a dual-layer composite material of the present disclosure (the dual-layer composite material formed by Example 1 shown above).

Or, the rats were divided into four groups, wherein the first group was a control group, the second group was treated with a commercial product, Seprafilm®, and the third group and the fourth group both were treated with a dual-layer composite material of the present disclosure (the dual-layer composite material formed by Example 1 shown above).

Or, the rats were divided into six groups, wherein the first group, the second group, and the third group were control groups, and the fourth group, the fifth group, and the sixth group were all treated with a dual-layer composite material of the present disclosure (the dual-layer composite material formed by Example 2 shown above).

### 1.2 Anesthesia

Zoletil 50: 0.025 ml/100 g body weight (B.W.) + xylazine (Rompun): 0.025 ml/100 g body weight; intra-peritoneal (I.P.) injection

### 1.3 Shaving hairs and spraying ethanol

Hairs on the abdomen were shaved by an electric razor, and then the ethanol was sprayed thereon to perform a local sterilization to prevent infection.

### 1.4 Laparotomy

Abdomen of rat was cut upward from the second pair of teats near the anus to create a wound with a size of 3-4 cm

### 1.5 Creating wounds

### 1.5.1 Stitch buttons on right peritoneal wall

Stitch buttons were stitched on right peritoneal wall from the position corresponding to the second pair of teats for making a total of four buttons. The buttons were spaced at an interval of 0.5 cm. Each button has three knots thereon.

### 1.5.2 Incisions and sutures for left uterine horn

A 1 cm wound in the uterine horn near a position 1 cm above the uterus was cut and then sutured with three sutures.

### 1.5.3 Wound on left peritoneal wall

A wound with a diameter of about 2 cm and a depth of about 0.5-1 mm was made in the left peritoneal wall.

### 1.6 Use of adhesion prevention material

After the redundant liquid was removed from the wound, a adhesion prevention membrane with a size of 2 cm x 2 cm (Seprafilm® or a dual-layer composite material of the present disclosure) was applied by dry forceps to cover the wound (there was no cover for the control group). The adhesion prevention membrane had to completely cover the wound to provide complete protection.

### 1.7 Suture for wound

Suture was performed by 3-0 black silk sutures. The muscle layer was sutured by continuous suture while the epidermal layer was sutured by noncontinuous suture. Finally, tincture of iodine was smeared on the wound on the epidermal layer, and the rat waited to be revived.

### 1.8 Evaluation of efficacy of adhesion prevention membranes

14 days after the operation, post-operation adhesion was evaluated by operators with double check, and adhesion evaluation table was made according to Linsky scoring system (see J. Reprod. Med., 32, 17-20, 1987 and Human Reproduction Vol. 18, No.8 pp.1703-1706, 2003 which are incorporated herein by reference). Also, the tissue specimens were collected for pathological evaluation of tissue repair.

### Scoring standard for the Linsky scoring system is described below

### (1) Adhesion extent

0= no adhesions
1= 20% of the traumatized area
2= 50% of the traumatized area
3= total involvement

### (2) Adhesion severity

0= no resistance for separation
0.5= some resistance for separation (moderate force is required)
1= sharp dissection is needed

### 1.9 Notice

1.9.1 Treatment time for each rat must be the same (25-30 minutes/rat)
1.9.2 All operations have to be performed by the same person to ensure consistency.

### 2. Experimental results

### (1) Animal experiments for the material from Example 1

Three independent experiments were performed.

### (i) First experiment

Positions and manner for creating wounds in the first experiment comprised stitch buttons on right peritoneal wall, electro-coagulation on right uterine horn and incisions and sutures for left uterine horn. After the operations, the rats were observed for 28 days, and 14 days after the operations, wound adhesion evaluation was performed on the rats in each group. The rat numbered 1-1 died after the operations. Evaluation results for wound adhesion for each group are shown in Table 1. According to Table 1, as compared to the commercial product, the dual-layer composite material of the present disclosure has a better anti-adhesion effect.

**Table 1: Adhesion evaluation for the first experiment**

| Rat number | Stitch buttons on right peritoneal wall | | | Electro-coagulation on right uterine horn | | | Incisions and sutures for left uterine horn | | | Body weight (g) | |
|---|---|---|---|---|---|---|---|---|---|---|---|
| | E | S | Total | E | S | Total | E | S | Total | Before operations | After operations |
| First group (control group) | | | | | | | | | | | |
| 1-2 | 1.00 | 0.50 | 1.50 | 0.00 | 0.00 | 0.00 | 3.00 | 1.00 | 4.00 | 240.00 | 250.00 |
| 1-3 | 3.00 | 1.00 | 4.00 | 3.00 | 1.00 | 4.00 | 3.00 | 1.00 | 4.00 | 240.00 | 271.00 |
| 1-4 | 3.00 | 1.00 | 4.00 | 3.00 | 1.00 | 4.00 | 3.00 | 1.00 | 4.00 | 240.00 | 284.00 |
| Mean | 2.33 | 0.83 | 3.17 | 2.00 | 0.67 | 2.67 | 3.00 | 1.00 | 4.00 | 240.00 | 268.33 |
| Standard deviation | 0.94 | 0.24 | 1.18 | 1.41 | 0.47 | 1.89 | 0.00 | 0.00 | 0.00 | 0.00 | 14.01 |

| Second group (Seprafilm®) | | | | | | | | | | | |
|---|---|---|---|---|---|---|---|---|---|---|---|
| 2-1 | 0.00 | 0.00 | 0.00 | 2.00 | 0.50 | 2.50 | 2.00 | 1.00 | 3.00 | 240.00 | 282.00 |
| 2-2 | 3.00 | 1.00 | 4.00 | 3.00 | 1.00 | 4.00 | 3.00 | 1.00 | 4.00 | 240.00 | 266.00 |
| 2-3 | 2.00 | 0.50 | 2.50 | 2.00 | 0.50 | 2.50 | 2.00 | 1.00 | 3.00 | 240.00 | 277.00 |
| Mean | 1.67 | 0.50 | 2.17 | 2.33 | 0.67 | 3.00 | 2.33 | 1.00 | 3.33 | 240.00 | 275.00 |
| Standard deviation | 1.25 | 0.41 | 1.65 | 0.47 | 0.24 | 0.71 | 0.47 | 0.00 | 0.47 | 0.00 | 6.68 |

| Third group (Example 1) | | | | | | | | | | | |
|---|---|---|---|---|---|---|---|---|---|---|---|
| 3-1 | 2.00 | 0.50 | 2.50 | 1.00 | 0.00 | 1.00 | 2.00 | 1.00 | 3.00 | 240.00 | 281.00 |
| 3-2 | 1.00 | 0.50 | 1.50 | 2.00 | 0.50 | 2.50 | 3.00 | 1.00 | 4.00 | 240.00 | 283.00 |
| 3-3 | 2.00 | 1.00 | 3.00 | 0.00 | 0.00 | 0.00 | 2.00 | 1.00 | 3.00 | 240.00 | 272.00 |
| Mean | 1.67 | 0.67 | 2.33 | 1.00 | 0.17 | 1.17 | 2.33 | 1.00 | 3.33 | 240.00 | 278.67 |
| Standard deviation | 0.47 | 0.24 | 0.62 | 0.82 | 0.24 | 1.03 | 0.47 | 0.00 | 0.47 | 0.00 | 4.78 |

| | | | | | | | | | | | |
|---|---|---|---|---|---|---|---|---|---|---|---|
| E : Adhesion extent S: Adhesion severity | | | | | | | | | | | |

### (ii) Second experiment

Positions and manner for creating wounds in the second experiment comprised stitch buttons on right peritoneal wall, electro-coagulation on left peritoneal wall and incisions and sutures for left uterine horn. After operations, the rats were observed for 14 days, and 14 days after operations, wound adhesion evaluation was performed on the rats in each group. The rat numbered 3-1 died after the operations. Evaluation results for wound adhesion for each group are shown in Table 2. According to Table 2, as compared to the commercial product, the dual-layer composite material of the present disclosure has a better anti-adhesion effect.

**Table 2: Adhesion evaluation for the second experiment**

| Rat number | Stitch buttons on right peritoneal wall | | | Electro-coagulation on left peritoneal wall | | | Incisions and sutures for left uterine horn | | | Body weight (g) | |
|---|---|---|---|---|---|---|---|---|---|---|---|
| | E | S | Total | E | S | Total | E | S | Total | Before operations | After operations |
| First group (control group) | | | | | | | | | | | |
| 1-1 | 3.00 | 1.00 | 4.00 | 2.00 | 0.50 | 2.50 | 3.00 | 1.00 | 4.00 | 250.00 | 260.50 |
| 1-2 | 3.00 | 1.00 | 4.00 | 0.00 | 0.00 | 0.00 | 3.00 | 1.00 | 4.00 | 248.20 | 265.00 |
| 1-3 | 3.00 | 1.00 | 4.00 | 0.00 | 0.00 | 0.00 | 3.00 | 1.00 | 4.00 | 251.70 | 265.00 |
| Mean | 3.00 | 1.00 | 4.00 | 0.67 | 0.17 | 0.83 | 3.00 | 1.00 | 4.00 | 249.97 | 263.50 |
| Standard deviation | 0.00 | 0.00 | 0.00 | 0.94 | 0.24 | 1.18 | 0.00 | 0.00 | 0.00 | 1.43 | 2.12 |

| Second group (Seprafilm®) | | | | | | | | | | | |
|---|---|---|---|---|---|---|---|---|---|---|---|
| 2-1 | 3.00 | 1.00 | 4.00 | 0.00 | 0.00 | 0.00 | 2.00 | 1.00 | 3.00 | 250.00 | 289.00 |
| 2-2 | 3.00 | 1.00 | 4.00 | 0.00 | 0.00 | 0.00 | 2.00 | 0.50 | 2.50 | 246.80 | 267.00 |
| 2-3 | 3.00 | 0.50 | 3.50 | 0.00 | 0.00 | 0.00 | 3.00 | 1.00 | 4.00 | 266.10 | 275.00 |
| Mean | 3.00 | 0.83 | 3.83 | 0.00 | 0.00 | 0.00 | 2.33 | 0.83 | 3.17 | 254.30 | 277.00 |
| Standard d eviation | 0.00 | 0.24 | 0.24 | 0.00 | 0.00 | 0.00 | 0.47 | 0.24 | 0.62 | 8.45 | 9.09 |

| Third group (Example 1) | | | | | | | | | | | |
|---|---|---|---|---|---|---|---|---|---|---|---|
| 3-1 | Dead | | | | | | | | | | |
| 3-2 | 2.00 | 0.50 | 2.50 | 0.00 | 0.00 | 0.00 | 2.00 | 1.00 | 3.00 | 249.10 | 253.00 |
| 3-3 | 1.00 | 0.50 | 1.50 | 0.00 | 0.00 | 0.00 | 2.00 | 1.00 | 3.00 | 264.60 | 254.00 |
| Mean | 1.50 | 0.50 | 2.00 | 0.00 | 0.00 | 0.00 | 2.00 | 1.00 | 3.00 | 256.85 | 253.50 |
| Standard deviation | 0.50 | 0.00 | 0.50 | 0.00 | 0.00 | 0.00 | 0.00 | 0.00 | 0.00 | 7.75 | 0.50 |

| | | | | | | | | | | | |
|---|---|---|---|---|---|---|---|---|---|---|---|
| E : Adhesion extent S: Adhesion severity | | | | | | | | | | | |

### (ii) Third experiment

Positions and manner for creating wounds in the third experiment comprised stitch buttons on right peritoneal wall, electro-coagulation on left peritoneal wall and incisions and sutures for left uterine horn. After the operations, the rats were observed for 14 days, and 14 days after the operations, wound adhesion evaluation was performed on the rats in each group. Evaluation results for wound adhesion for each group are shown in Table 3. According to Table 3, as compared to the commercial product, the dual-layer composite material of the present disclosure has a better anti-adhesion effect.

**Table 3: Adhesion evaluation for the third experiment**

| Rat number | Stitch buttons on right peritoneal wall | | | Electro-coagulation on left peritoneal wall | | | Incisions and sutures for left uterine horn | | | Body weight (g) | |
|---|---|---|---|---|---|---|---|---|---|---|---|
| | E | S | Total | E | S | Total | E | S | Total | Before operations | After operations |
| First group (control group) | | | | | | | | | | | |
| 1-1' | 3.00 | 1.00 | 4.00 | 0.00 | 0.00 | 0.00 | 3.00 | 1.00 | 4.00 | 265.66 | 261.00 |
| 1-2' | 3.00 | 1.00 | 4.00 | 1.00 | 0.50 | 1.50 | 3.00 | 1.00 | 4.00 | 255.00 | 281.00 |
| 1-3' | 3.00 | 1.00 | 4.00 | 3.00 | 1.00 | 4.00 | 3.00 | 1.00 | 4.00 | 255.00 | 265.00 |
| Mean | 3.00 | 1.00 | 4.00 | 1.33 | 0.50 | 1.83 | 3.00 | 1.00 | 4.00 | 258.55 | 269.00 |
| Standard deviation | 0.00 | 0.00 | 0.00 | 1.25 | 0.41 | 1.65 | 0.00 | 0.00 | 0.00 | 5.03 | 8.64 |

| Second group (Seprafilm®) | | | | | | | | | | | |
|---|---|---|---|---|---|---|---|---|---|---|---|
| 2-1' | 3.00 | 1.00 | 4.00 | 0.00 | 0.00 | 0.00 | 3.00 | 1.00 | 4.00 | 263.67 | 258.00 |
| 2-2' | 3.00 | 1.00 | 4.00 | 3.00 | 1.00 | 4.00 | 3.00 | 1.00 | 4.00 | 258.00 | 300.00 |
| 2-3' | 3.00 | 1.00 | 4.00 | 0.00 | 0.00 | 0.00 | 3.00 | 1.00 | 4.00 | 260.00 | 286.00 |
| Mean | 3.00 | 1.00 | 4.00 | 1.00 | 0.33 | 1.33 | 3.00 | 1.00 | 4.00 | 260.56 | 281.33 |
| Standard deviation | 0.00 | 0.00 | 0.00 | 1.41 | 0.47 | 1.89 | 0.00 | 0.00 | 0.00 | 2.35 | 17.46 |

| Third group (Example 1) | | | | | | | | | | | |
|---|---|---|---|---|---|---|---|---|---|---|---|
| 3-1' | 3.00 | 0.50 | 3.50 | 0.00 | 0.00 | 0.00 | 0.00 | 0.00 | 0.00 | 266.13 | 297.00 |
| 3-2' | 3.00 | 0.50 | 3.50 | 3.00 | 1.00 | 4.00 | 0.00 | 0.00 | 0.00 | 260.20 | 269.00 |
| 3-3' | 3.00 | 0.50 | 3.50 | 3.00 | 1.00 | 4.00 | 0.00 | 0.00 | 0.00 | 259.60 | 284.00 |
| Mean | 3.00 | 0.50 | 3.50 | 2.00 | 0.67 | 2.67 | 0.00 | 0.00 | 0.00 | 261.98 | 283.33 |
| Standard deviation | 0.00 | 0.00 | 0.00 | 1.41 | 0.47 | 1.89 | 0.00 | 0.00 | 0.00 | 2.95 | 11.44 |

| Fourth group (Example 1) | | | | | | | | | | | |
|---|---|---|---|---|---|---|---|---|---|---|---|
| 4-1' | 3.00 | 0.50 | 3.50 | 3.00 | 1.00 | 4.00 | 0.00 | 0.00 | 0.00 | 264.47 | 291.00 |
| 4-2' | 3.00 | 0.50 | 3.50 | 0.00 | 0.00 | 0.00 | 1.00 | 0.50 | 1.50 | 266.00 | 271.00 |
| 4-3' | 1.00 | 0.50 | 1.50 | 1.00 | 0.50 | 1.50 | 0.00 | 0.00 | 0.00 | 262.60 | 276.00 |
| Mean | 2.33 | 0.50 | 2.83 | 1.33 | 0.50 | 1.83 | 0.33 | 0.17 | 0.50 | 264.36 | 279.33 |
| Standard deviation | 0.94 | 0.00 | 0.94 | 1.25 | 0.41 | 1.65 | 0.47 | 0.24 | 0.71 | 1.39 | 8.50 |

| | | | | | | | | | | | |
|---|---|---|---|---|---|---|---|---|---|---|---|
| E : Adhesion extent S: Adhesion severity | | | | | | | | | | | |

### (2) Animal experiments for the material from Example 2

Positions and manner for creating wounds comprised electro-coagulation on left peritoneal wall and incisions and sutures for left uterine horn. After operations, the rats were observed for 14 days, and 14 days after operations, wound adhesion evaluation was performed on the rat in each group. Evaluation results for wound adhesion for each group are shown in Table 4. According to Table 4, it is known that the dual-layer composite material of the present disclosure has a better anti-adhesion effect.

**Table 4: Adhesion evaluation**

| | Electro-coagulation on left peritoneal wall | | Incisions and sutures for left uterine horn | | Body weight (g) | |
|---|---|---|---|---|---|---|
| | E | S | E | S | Before operations | After operations |
| First group (control group) | 3 | 1 | 3 | 1 | 315.3 | 312.8 |
| Second group (control group) | 3 | 1 | 3 | 1 | 311.8 | 309.3 |
| Third group (control group) | 0 | 0 | 3 | 1 | 299.7 | 307.8 |
| Fourth group (Example 2) | 0 | 0 | 2 | 0.5 | 346.1 | 342.3 |
| Fifth group (Example 2) | 0 | 0 | 0 | 0 | 311.4 | 287.3 |
| Sixth group (Example 2) | 1 | 0.5 | 1 | 0.5 | 289.7 | 296.5 |

| | | | | | | |
|---|---|---|---|---|---|---|
| E : Adhesion extent S: Adhesion severity | | | | | | |

## Claims

1. A method for forming a dual-layer composite material, wherein the dual-layer composite material is a biomedical material, comprising:
coating a barrier-film layer-forming material onto a surface of a porous scaffold layer to form a dual-layer intermediate product; and
drying the dual-layer intermediate product to form a dual-layer composite material which comprises the porous scaffold layer and a barrier film layer, wherein the porous scaffold layer and the barrier film layer are inseparable from each other, and
wherein a method for forming the porous scaffold layer comprises:
mixing at least one first biodegradable polymer with a first solvent under a stirring speed of 3500-12000 rpm, at 4-10°C, for 90-180 minutes, to form a slurry, wherein the at least one first biodegradable polymer is selected from a group consisting of collagen, gelatin, chitosan, and a combination of collagen, gelatin or chitosan, and hyaluronic acid;
placing the slurry into a mold and freezing the slurry;
performing a lyophilization procedure on the slurry to remove water molecules and obtain a scaffold body; and
performing a vacuum heating procedure on the scaffold body to dehydrate and crosslink the scaffold body to form the porous scaffold layer, and
wherein a method for forming the barrier-film layer-forming material comprises:
mixing a second biodegradable polymer with a second solvent and then letting it stand to form a gel, wherein the second biodegradable polymer is selected from a group consisting of collagen, gelatin, chitosan and hyaluronic acid; and
stirring the gel to a homogeneous stage to form the barrier-film layer-forming material.

2. The method for forming a dual-layer composite material as claimed in claim 1, wherein the at least one first biodegradable polymer accounts for 1.0-2.0 wt% of the slurry.

3. The method for forming a dual-layer composite material as claimed in claim 1, wherein the at least one first biodegradable polymer is collagen.

4. The method for forming a dual-layer composite material as claimed in claim 3, wherein the collagen is type I collagen.

5. The method for forming a dual-layer composite material as claimed in claim 1, wherein the at least one first biodegradable polymer is a combination of collagen, gelatin or chitosan, and hyaluronic acid.

6. The method for forming a dual-layer composite material as claimed in claim 5, before the step of mixing at least one first biodegradable polymer with a first solvent under a stirring speed of 3500-12000 rpm, at 4-10°C, for90-180 minutes, to form a slurry, further comprising:
mixing the collagen, gelatin or chitosan with the first solvent under a stirring speed of 3500-12000 rpm, at 4-10°C, for 90-180 minutes, previously, to form a mixture; and
pouring a solution of the hyaluronic acid into the mixture.

7. The method for forming a dual-layer composite material as claimed in claim 1, wherein the at least one first biodegradable polymer is a combination of collagen and hyaluronic acid.

8. The method for forming a dual-layer composite material as claimed in claim 7, wherein the collagen is type I collagen.

9. The method for forming a dual-layer composite material as claimed in claim 7, wherein a weight ratio of the collagen to the hyaluronic acid is 90-99.9:10-0.1.

10. The method for forming a dual-layer composite material as claimed in claim 1, wherein the first solvent comprises water, isopropanol or acetic acid.

11. The method for forming a dual-layer composite material as claimed in claim 1, wherein in the step of placing the slurry into a mold and freezing the slurry, the slurry is frozen at -40 to -20°C.

12. The method for forming a dual-layer composite material as claimed in claim 1, wherein a pressure in the lyophilization procedure is 3.99-26.6 Pa (30-200 mTorr).

13. The method for forming a dual-layer composite material as claimed in claim 12, wherein the lyophilization procedure comprises:
at -40 to -20°C, freezing the slurry for3-6 hours;
at -40 to 10°C, freezing the slurry for12-16 hours; and
placing the slurry at20 to 30°C, for 3-6 hours.

14. The method for forming a dual-layer composite material as claimed in claim 1, wherein a pressure in the vacuum heating procedure is 3.99-26.6 Pa (30-200 mTorr).

15. The method for forming a dual-layer composite material as claimed in claim 1, wherein a temperature in the vacuum heating procedure is 80-110 °C.

16. The method for forming a dual-layer composite material as claimed in claim 1, wherein time for the vacuum heating procedure is 12-24 hours.

17. The method for forming a dual-layer composite material as claimed in claim 1, between the step of mixing at least one first biodegradable polymer with a first solvent under a stirring speed of 3500-12000 rpm, at 4-10°C, for 90-180 minutes, to form a slurry, and the step of placing the slurry into a mold and freezing the slurry, further comprising a step of removing gas in the slurry.

18. The method for forming a dual-layer composite material as claimed in claim 1, wherein the second biodegradable polymer accounts for 3-10% w/v of the gel.

19. The method for forming a dual-layer composite material as claimed in claim 1, wherein the second biodegradable polymer is hyaluronic acid.

20. The method for forming a dual-layer composite material as claimed in claim 1, wherein the second biodegradable polymer is collagen.

21. The method for forming a dual-layer composite material as claimed in claim 20, wherein the collagen is type I collagen.

22. The method for forming a dual-layer composite material as claimed in claim 1, wherein the second solvent comprises water or acetic acid.

23. The method for forming a dual-layer composite material as claimed in claim 1, wherein in the step of mixing a second biodegradable polymer with a second solvent and then letting it stand, with a standing time of 12-24 hours.

24. The method for forming a dual-layer composite material as claimed in claim 1, after the step of stirring the gel to a homogeneous stage, further comprising removing bubbles in the gel.

25. The method for forming a dual-layer composite material as claimed in claim 1, wherein the dual-layer intermediate product is dried at 15-30°C.

26. The method for forming a dual-layer composite material as claimed in claim 1, wherein the dual-layer intermediate product is dried for 12-24 hours.

27. The method for forming a dual-layer composite material as claimed in claim 1, wherein the dual-layer intermediate product is dried at a room temperature for 24 hours.

28. A dual-layer composite material formed by the method for forming a dual-layer composite material as claimed in claim 1, wherein the dual-layer composite material is in the form of a membrane or in the form of a 3-dimensional shape comprising a hollow part or channel with at least one opening.

29. The dual-layer composite material as claimed in claim 28, wherein the 3-dimensional shape is a globe shape, an oval-shape, a tube shape, a funnel shape or a cone shape.

30. The dual-layer composite material as claimed in claim 28, wherein the dual-layer composite material is in the form of a 3-dimensional shape comprising a hollow part or channel with at least one opening, and wherein the porous scaffold layer is located outside of the 3-dimensional shape while the barrier film layer is located inside of the 3-dimensional shape and encompasses the hollow part or channel, or wherein the barrier film layer is located outside of the 3-dimensional shape while the porous scaffold layer is located inside of the 3-dimensional shape and encompasses the hollow part or channel.

## Patentansprüche

1. Verfahren zum Bilden eines zweischichtigen Verbundmaterials, wobei das zweischichtige Verbundmaterial ein biomedizinisches Material ist, umfassend:
Beschichten eines schichtbildenden Barrierefilm-Materials auf eine Oberfläche einer porösen Gerüstschicht, wobei ein zweischichtiges Zwischenprodukt gebildet wird; und
Trocknen des zweischichtigen Zwischenprodukts, wobei ein zweischichtiges Verbundmaterial gebildet wird, welches die poröse Gerüstschicht und eine Barrierefilm-Schicht umfasst, wobei die poröse Gerüstschicht und die Barrierefilm-Schicht nicht voneinander getrennt werden können, und
wobei ein Verfahren zum Bilden der porösen Gerüstschicht umfasst:
Mischen von mindestens einem ersten bioabbaubaren Polymer mit einem ersten Lösungsmittel unter einer Rührgeschwindigkeit von 3500-12000 UpM bei 4-10°C 90-180 Minuten lang, wobei eine Aufschlämmung gebildet wird, wobei das mindestens eine erste bioabbaubare Polymer aus einer Gruppe ausgewählt ist, welche aus Kollagen, Gelatine, Chitosan, und einer Kombination von Kollagen, Gelatine oder Chitosan, und Hyaluronsäure besteht;
Platzieren der Aufschlämmung in einer Form und Gefrieren der Aufschlämmung;
Durchführen eines Lyophilisierungsverfahrens an der Aufschlämmung, wobei Wassermoleküle entfernt werden und ein Gerüstkörper erhalten wird; und
Durchführen eines Vakuum-Erwärmungsverfahrens an dem Gerüstkörper, um den Gerüstkörper zu dehydratisieren und zu vernetzen, wobei die poröse Gerüstschicht gebildet wird, und
wobei ein Verfahren zum Bilden des schichtbildenden Barrierefilm-Materials umfasst:
Mischen eines zweiten bioabbaubaren Polymers mit einem zweiten Lösungsmittel und dann Stehenlassen, wobei ein Gel gebildet wird, wobei das zweite bioabbaubare Polymer aus einer Gruppe ausgewählt ist, welche aus Kollagen, Gelatine, Chitosan und Hyaluronsäure besteht; und
Rühren des Gels bis zu einem homogenen Zustand, wobei das schichtbildende Barrierefilm-Material gebildet wird.

2. Verfahren zum Bilden eines zweischichtigen Verbundmaterials wie in Anspruch 1 beansprucht, wobei das mindestens eine erste bioabbaubare Polymer 1,0-2,0 Gew.-% der Aufschlämmung beträgt.

3. Verfahren zum Bilden eines zweischichtigen Verbundmaterials wie in Anspruch 1 beansprucht, wobei das mindestens eine erste bioabbaubare Polymer Kollagen ist.

4. Verfahren zum Bilden eines zweischichtigen Verbundmaterials wie in Anspruch 3 beansprucht, wobei das Kollagen Typ I-Kollagen ist.

5. Verfahren zum Bilden eines zweischichtigen Verbundmaterials wie in Anspruch 1 beansprucht, wobei das mindestens eine erste bioabbaubare Polymer eine Kombination von Kollagen, Gelatine oder Chitosan, und Hyaluronsäure ist.

6. Verfahren zum Bilden eines zweischichtigen Verbundmaterials wie in Anspruch 5 beansprucht, vor dem Schritt von Mischen von mindestens einem ersten bioabbaubaren Polymer mit einem ersten Lösungsmittel unter einer Rührgeschwindigkeit von 3500-12000 UpM bei 4-10°C 90-180 Minuten lang, wobei eine Aufschlämmung gebildet wird, ferner umfassend:
Mischen des Kollagens, der Gelatine oder des Chitosans mit dem ersten Lösungsmittel unter einer Rührgeschwindigkeit von 3500-12000 UpM bei 4-10°C 90-180 Minuten lang, vorher, wobei ein Gemisch gebildet wird; und
Gießen einer Lösung der Hyaluronsäure in das Gemisch.

7. Verfahren zum Bilden eines zweischichtigen Verbundmaterials wie in Anspruch 1 beansprucht, wobei das mindestens eine erste bioabbaubare Polymer eine Kombination von Kollagen und Hyaluronsäure ist.

8. Verfahren zum Bilden eines zweischichtigen Verbundmaterials wie in Anspruch 7 beansprucht, wobei das Kollagen Typ I-Kollagen ist.

9. Verfahren zum Bilden eines zweischichtigen Verbundmaterials wie in Anspruch 7 beansprucht, wobei ein Gewichtsverhältnis von dem Kollagen zu der Hyaluronsäure 90-99,9:10-0,1 beträgt.

10. Verfahren zum Bilden eines zweischichtigen Verbundmaterials wie in Anspruch 1 beansprucht, wobei das erste Lösungsmittel Wasser, Isopropanol oder Essigsäure umfasst.

11. Verfahren zum Bilden eines zweischichtigen Verbundmaterials wie in Anspruch 1 beansprucht, wobei im Schritt von Platzieren der Aufschlämmung in einer Form und Gefrieren der Aufschlämmung die Aufschlämmung bei -40 bis -20°C gefroren wird.

12. Verfahren zum Bilden eines zweischichtigen Verbundmaterials wie in Anspruch 1 beansprucht, wobei ein Druck in dem Lyophilisierungsverfahren 3,99-26,6 Pa (30-200 mTorr) beträgt.

13. Verfahren zum Bilden eines zweischichtigen Verbundmaterials wie in Anspruch 12 beansprucht, wobei das Lyophilisierungsverfahren umfasst:
Gefrieren der Aufschlämmung bei -40 bis -20°C 3-6 Stunden lang;
Gefrieren der Aufschlämmung bei -40 bis 10°C 12-16 Stunden lang; und
Platzieren der Aufschlämmung bei 20 bis 30°C 3-6 Stunden lang.

14. Verfahren zum Bilden eines zweischichtigen Verbundmaterials wie in Anspruch 1 beansprucht, wobei ein Druck in dem Vakuum-Erwärmungsverfahren 3,99-26,6 Pa (30-200 mTorr) beträgt.

15. Verfahren zum Bilden eines zweischichtigen Verbundmaterials wie in Anspruch 1 beansprucht, wobei eine Temperatur in dem Vakuum-Erwärmungsverfahren 80-110°C beträgt.

16. Verfahren zum Bilden eines zweischichtigen Verbundmaterials wie in Anspruch 1 beansprucht, wobei die Zeit für das Vakuum-Erwärmungsverfahren 12-24 Stunden beträgt.

17. Verfahren zum Bilden eines zweischichtigen Verbundmaterials wie in Anspruch 1 beansprucht, zwischen dem Schritt von Mischen von mindestens einem ersten bioabbaubaren Polymer mit einem ersten Lösungsmittel unter einer Rührgeschwindigkeit von 3500-12000 UpM bei 4-10°C 90-180 Minuten lang, wobei eine Aufschlämmung gebildet wird, und dem Schritt von Platzieren der Aufschlämmung in einer Form und Gefrieren der Aufschlämmung ferner umfassend einen Schritt von Entfernen von Gas in der Aufschlämmung.

18. Verfahren zum Bilden eines zweischichtigen Verbundmaterials wie in Anspruch 1 beansprucht, wobei das zweite bioabbaubare Polymer 3-10 % w/v des Gels beträgt.

19. Verfahren zum Bilden eines zweischichtigen Verbundmaterials wie in Anspruch 1 beansprucht, wobei das zweite bioabbaubare Polymer Hyaluronsäure ist.

20. Verfahren zum Bilden eines zweischichtigen Verbundmaterials wie in Anspruch 1 beansprucht, wobei das zweite bioabbaubare Polymer Kollagen ist.

21. Verfahren zum Bilden eines zweischichtigen Verbundmaterials wie in Anspruch 20 beansprucht, wobei das Kollagen Typ I-Kollagen ist.

22. Verfahren zum Bilden eines zweischichtigen Verbundmaterials wie in Anspruch 1 beansprucht, wobei das zweite Lösungsmittel Wasser oder Essigsäure umfasst.

23. Verfahren zum Bilden eines zweischichtigen Verbundmaterials wie in Anspruch 1 beansprucht, wobei im Schritt von Mischen eines zweiten bioabbaubaren Polymers mit einem zweiten Lösungsmittel und dann Stehenlassen eine Standzeit 12-24 Stunden beträgt.

24. Verfahren zum Bilden eines zweischichtigen Verbundmaterials wie in Anspruch 1 beansprucht, nach dem Schritt von Rühren des Gels bis zu einem homogenen Zustand ferner umfassend Entfernen von Blasen in dem Gel.

25. Verfahren zum Bilden eines zweischichtigen Verbundmaterials wie in Anspruch 1 beansprucht, wobei das zweischichtige Zwischenprodukt bei 15-30°C getrocknet wird.

26. Verfahren zum Bilden eines zweischichtigen Verbundmaterials wie in Anspruch 1 beansprucht, wobei das zweischichtige Zwischenprodukt 12-24 Stunden lang getrocknet wird.

27. Verfahren zum Bilden eines zweischichtigen Verbundmaterials wie in Anspruch 1 beansprucht, wobei das zweischichtige Zwischenprodukt bei einer Raumtemperatur 24 Stunden lang getrocknet wird.

28. Zweischichtiges Verbundmaterial, gebildet durch das Verfahren zum Bilden eines zweischichtigen Verbundmaterials wie in Anspruch 1 beansprucht, wobei das zweischichtige Verbundmaterial in der Form einer Membran oder in der Form eines dreidimensionalen Gebildes, umfassend einen Hohlteil oder Kanal mit mindestens einer Öffnung, vorliegt.

29. Zweischichtiges Verbundmaterial wie in Anspruch 28 beansprucht, wobei das dreidimensionale Gebilde ein kugelförmiges Gebilde, ein ovales Gebilde, ein rohrförmiges Gebilde, ein trichterförmiges Gebilde oder ein kegelförmiges Gebilde ist.

30. Zweischichtiges Verbundmaterial wie in Anspruch 28 beansprucht, wobei das zweischichtige Verbundmaterial in der Form eines dreidimensionalen Gebildes, umfassend einen Hohlteil oder Kanal mit mindestens einer Öffnung, vorliegt, und wobei die poröse Gerüstschicht außen an dem dreidimensionalen Gebilde lokalisiert ist, während die Barrierefilm-Schicht innen an dem dreidimensionalen Gebilde lokalisiert ist und den Hohlteil oder Kanal umfasst, oder wobei die Barrierefilm-Schicht außen an dem dreidimensionalen Gebilde lokalisiert ist, während die poröse Gerüstschicht innen an dem dreidimensionalen Gebilde lokalisiert ist und den Hohlteil oder Kanal umfasst.

## Revendications

1. Procédé de formation d'un matériau composite bicouche, dans lequel le matériau composite bicouche est un matériau biomédical, comprenant :
l'enduction d'un matériau de formation de couche de film barrière sur une surface d'une couche d'échafaudage poreuse pour former un produit intermédiaire bicouche ; et
le séchage du produit intermédiaire bicouche pour former un matériau composite bicouche qui comprend la couche d'échafaudage poreuse et une couche de film barrière, dans lequel la couche d'échafaudage poreuse et la couche de film barrière sont inséparables l'une de l'autre, et
dans lequel un procédé de formation de la couche d'échafaudage poreuse comprend :
le mélange d'au moins un premier polymère biodégradable avec un premier solvant à une vitesse d'agitation de 3500-12000 tr/min, à 4-10 °C, pendant 90-180 minutes, pour former une suspension, dans lequel l'au moins un premier polymère biodégradable est choisi dans un groupe constitué de collagène, de gélatine, de chitosane et d'une combinaison de collagène, de gélatine ou de chitosane et d'acide hyaluronique ;
le placement de la suspension dans un moule et la congélation de la suspension ;
la réalisation d'une procédure de lyophilisation sur la suspension pour éliminer les molécules d'eau et obtenir un corps d'échafaudage ; et
la réalisation d'une procédure de chauffage sous vide sur le corps d'échafaudage pour déshydrater et réticuler le corps d'échafaudage pour former la couche d'échafaudage poreuse, et
dans lequel un procédé de formation du matériau de formation de couche de film barrière comprend :
le mélange d'un second polymère biodégradable avec un second solvant et ensuite le fait de le laisser reposer pour former un gel, dans lequel le second polymère biodégradable est choisi dans un groupe constitué de collagène, de gélatine, de chitosane et d'acide hyaluronique ; et
l'agitation du gel jusqu'à un stade homogène pour former le matériau de formation de couche de film barrière.

2. Procédé de formation d'un matériau composite bicouche selon la revendication 1, dans lequel l'au moins un premier polymère biodégradable représente 1,0-2,0 % en poids de la suspension.

3. Procédé de formation d'un matériau composite bicouche selon la revendication 1, dans lequel l'au moins un premier polymère biodégradable est du collagène.

4. Procédé de formation d'un matériau composite bicouche selon la revendication 3, dans lequel le collagène est du collagène de type I.

5. Procédé de formation d'un matériau composite bicouche selon la revendication 1, dans lequel l'au moins un premier polymère biodégradable est une combinaison de collagène, de gélatine ou de chitosane et d'acide hyaluronique.

6. Procédé de formation d'un matériau composite bicouche selon la revendication 5, avant l'étape de mélange d'au moins un premier polymère biodégradable avec un premier solvant à une vitesse d'agitation de 3500-12000 tr/min, à 4-10 °C, pendant 90-180 minutes, pour former une suspension, comprenant en outre :
le mélange du collagène, de la gélatine ou du chitosane avec le premier solvant à une vitesse d'agitation de 3500-12000 tr/min, à 4-10 °C, pendant 90-180 minutes, préalablement, pour former un mélange ; et
le versement d'une solution de l'acide hyaluronique dans le mélange.

7. Procédé de formation d'un matériau composite bicouche selon la revendication 1, dans lequel l'au moins un premier polymère biodégradable est une combinaison de collagène et d'acide hyaluronique.

8. Procédé de formation d'un matériau composite bicouche selon la revendication 7, dans lequel le collagène est du collagène de type I.

9. Procédé de formation d'un matériau composite bicouche selon la revendication 7, dans lequel un rapport en poids du collagène sur l'acide hyaluronique est de 90-99,9 : 10-0,1.

10. Procédé de formation d'un matériau composite bicouche selon la revendication 1, dans lequel le premier solvant comprend de l'eau, de l'isopropanol ou de l'acide acétique.

11. Procédé de formation d'un matériau composite bicouche selon la revendication 1, dans lequel dans l'étape de placement de la suspension dans un moule et de congélation de la suspension, la suspension est congelée à -40 à -20 °C.

12. Procédé de formation d'un matériau composite bicouche selon la revendication 1, dans lequel une pression dans la procédure de lyophilisation est de 3,99-26,6 Pa (30-200 mTorr).

13. Procédé de formation d'un matériau composite bicouche selon la revendication 12, dans lequel la procédure de lyophilisation comprend :
à -40 à -20 °C, la congélation de la suspension pendant 3-6 heures ;
à -40 à 10 °C, la congélation de la suspension pendant 12-16 heures ; et
le placement de la suspension à 20 à 30 °C, pendant 3-6 heures.

14. Procédé de formation d'un matériau composite bicouche selon la revendication 1, dans lequel une pression dans la procédure de chauffage sous vide est de 3,99-26,6 Pa (30-200 mTorr).

15. Procédé de formation d'un matériau composite bicouche selon la revendication 1, dans lequel une température dans la procédure de chauffage sous vide est de 80-110 °C.

16. Procédé de formation d'un matériau composite bicouche selon la revendication 1, dans lequel le temps pour la procédure de chauffage sous vide est de 12-24 heures.

17. Procédé de formation d'un matériau composite bicouche selon la revendication 1, entre l'étape de mélange d'au moins un premier polymère biodégradable avec un premier solvant à une vitesse d'agitation de 3500-12000 tr/min, à 4-10 °C, pendant 90-180 minutes, pour former une suspension, et l'étape de placement de la suspension dans un moule et de congélation de la suspension, comprenant en outre une étape d'élimination du gaz dans la suspension.

18. Procédé de formation d'un matériau composite bicouche selon la revendication 1, dans lequel le second polymère biodégradable représente 3-10 % en poids/volume du gel.

19. Procédé de formation d'un matériau composite bicouche selon la revendication 1, dans lequel le second polymère biodégradable est de l'acide hyaluronique.

20. Procédé de formation d'un matériau composite bicouche selon la revendication 1, dans lequel le second polymère biodégradable est du collagène.

21. Procédé de formation d'un matériau composite bicouche selon la revendication 20, dans lequel le collagène est du collagène de type I.

22. Procédé de formation d'un matériau composite bicouche selon la revendication 1, dans lequel le second solvant comprend de l'eau ou de l'acide acétique.

23. Procédé de formation d'un matériau composite bicouche selon la revendication 1, dans lequel dans l'étape de mélange d'un second polymère biodégradable avec un second solvant et ensuite le laisser reposer, avec un temps de repos de 12-24 heures.

24. Procédé de formation d'un matériau composite bicouche selon la revendication 1, après l'étape d'agitation du gel jusqu'à un stade homogène, comprenant en outre l'élimination des bulles dans le gel.

25. Procédé de formation d'un matériau composite bicouche selon la revendication 1, dans lequel le produit intermédiaire bicouche est séché à 15-30 °C.

26. Procédé de formation d'un matériau composite bicouche selon la revendication 1, dans lequel le produit intermédiaire bicouche est séché pendant 12-24 heures.

27. Procédé de formation d'un matériau composite bicouche selon la revendication 1, dans lequel le produit intermédiaire bicouche est séché à température ambiante pendant 24 heures.

28. Matériau composite bicouche formé par le procédé de formation d'un matériau composite bicouche selon la revendication 1, dans lequel le matériau composite bicouche est sous la forme d'une membrane ou sous la forme d'une forme tridimensionnelle comprenant une partie creuse ou un canal avec au moins une ouverture.

29. Matériau composite bicouche selon la revendication 28, dans lequel la forme tridimensionnelle est une forme de globe, une forme ovale, une forme de tube, une forme d'entonnoir ou une forme de cône.

30. Matériau composite bicouche selon la revendication 28, dans lequel le matériau composite bicouche est sous la forme d'une forme tridimensionnelle comprenant une partie creuse ou un canal avec au moins une ouverture, et dans lequel la couche d'échafaudage poreuse est située à l'extérieur de la forme tridimensionnelle alors que la couche de film barrière est située à l'intérieur de la forme tridimensionnelle et englobe la partie creuse ou le canal, ou dans lequel la couche de film barrière est située à l'extérieur de la forme tridimensionnelle alors que la couche d'échafaudage poreuse est située à l'intérieur de la forme tridimensionnelle et englobe la partie creuse ou le canal.
